(19) Europäisches Patentamt
European Patent Office
Office européen des brevets

(11) Publication number: **0 159 915**
**A2**

(12) **EUROPEAN PATENT APPLICATION**

(21) Application number: **85302697.9**

(22) Date of filing: **17.04.85**

(51) Int. Cl.[4]: **C 07 C 25/28**, C 07 C 17/04, C 07 C 17/14, C 08 F 12/16

(30) Priority: **19.04.84 IL 71576**

(43) Date of publication of application: **30.10.85**
**Bulletin 85/44**

(84) Designated Contracting States: **AT BE CH DE FR GB IT LI LU NL SE**

(71) Applicant: **YISSUM RESEARCH AND DEVELOPMENT COMPANY OF THE HEBREW UNIVERSITY OF JERUSALEM, 46, Jabotinsky Street P.O. box 4279, Jerusalem 91042 (IL)**

(72) Inventor: **Wiener, Harold, 710/18 NaHagay Haprodot Street, Jerusalem (IL)**
Inventor: **Neumann, Ronny, 20/19 Bar Kochba Street, Jerusalem (IL)**
Inventor: **Sasson, Yoel, 30 Palmach Street, Jerusalem (IL)**

(74) Representative: **Bizley, Richard Edward et al, BOULT, WADE & TENNANT 27 Furnival Street, London EC4A 1PQ (GB)**

(54) **Process for making ring-halogenated styrenes and their use.**

(57) The invention provides a process for preparing a ring-halogenated styrene monomer comprising hearing an α-bromoethyl halobenzene at a temperature in the range of about 100-250°C in the presence of a polymerization inhibitor whereby a corresponding ring-halogenated styrene and hydrobromic acid are formed.

EP 0 159 915 A2

PROCESS FOR MAKING RING-HALOGENATED STYRENES AND THEIR USE

The present invention relates to a process for preparing ring-halogenated styrene monomers of high purity.

Styrene based polymers make up a very important part of the plastic industry. The polymeric substances are useful in the production of molded articles, latex paints, synthetic rubbers, coatings and also have proven useful as intermediates for the plastic industry.

Polymers having styrene as part of their structural make-up are for example, polystyrene, styrene-butadiene, copolymers, ABS, SAN, thermosetting polyester resins and copolymers of styrene with acrylate and maleic monomers. One great deficiency of these styrene based polymers, however, is their flammability, and there is an ever increasing awareness and interest in providing polymers which possess flame retardance. Nuclear halogenated substituted styrenes, have been known as flame retardants, particularly mono, di and tri bromo and chlorostyrenes One particular method of introducing flame retardance to styrene based polymers consists in substituting, or at least partially substituting, ring-halogenated styrene for styrene. This method of inducing flame retardance was disclosed in the literature and in patents but was never commercialized primarily because of the uneconomical method for preparing these ring halogenated styrenes.

It is well known that styrene may be formed by splitting hydrogen halide from a halo-ethylbenzene, and several methods for carrying out such reactions have been proposed. The dehydrohalogenation has been accomplished by pyrolyzing the halo-ethylbenzene at temperatures between 500-725°C

as reported in U.S. Pat. No. 1687903 to yield styrene regardless of whether the haloethylbenzene reactant contains its halogen in the ethyl group or in its nucleus. However, the same patent shows that the pyrolysis results in tar formation.

Styrene has been produced from α-chloroethylbenzene by heating the latter with an agent such as pyridine, which removes the hydrogen chloride split from pyrolysis, but the expense of using pyridine in the large proportions required renders this method poorly suited to commercial practice.

A third general method for producing styrene from side chain halogenated ethylbenzene has comprised heating the latter at a temperature of 150-175°C with a catalyst which is effective for promoting dehydrohalogenation of the haloethylbenzene and distilling off the styrene during or subsequent to the reaction.

Natelson (J.Ind.Eng.Chem. 25, 1391 (1933) has studied such catalytic dehydrohalogenation processes using a wide variety of catalysts and also testing certain variations in operating procedures, but the maximum yield reported was 65% of the theoretical, and a large amount of dark coloured material which could not be steam distilled was obtained.

R.R. Dreisbach and J.Day claimed in U.S. Patent No. 2295007 that when introducing both a catalyst and the α-haloethylbenzene gradually into a heated chamber at 200-350°C, while distilling styrene from the latter, styrene may be produced in yields far higher than have previously been obtainable with the same catalyst.

Most of the published commercial methods for making nuclear halogenated styrenes depend on the catalytic halogenation of the ethylbenzene nucleus followed by halogenation of the ethyl group at elevated temperatures giving halogenated ethylhalobenzenes, which are subsequently dehydrohalogenated to give the vinylhalobenzenes.

Thus, U.S. Pat. No. 2432737 discloses a process for preparing dichlorostyrenes by dehydrohalogenation of a mixture of α and β-chlorohalobenzenes on passing the mixture over silica gel with steam at 275°C. The β-compound is separated from the product by distillation and then dehydrohalogenated at 300-550°C. with steam over a metal silicate catalyst. This process is very complex, involving two dehydrohalogenation steps and involves difficult separations by fractional distillation.

An improvement of the above was disclosed in U.S. Patent No. 2485524. Chemically the process is similar to that described above with the main feature being the use of calcium sulphate as a dehydrohalogenation catalyst which dehydrohalogenates both α- and β-chloroethylhalobenzenes simultaneously in the presence of steam at 300-450°C. A disadvantage common to both the above processes is that the chlorination of ethylhalobenzenes must be stopped considerably short of the theoretical yield in order to avoid disubstitution in the ethyl group thus necessitating a fractional distillation to separate and recycle the unreacted ethylhalobenzene. This fractionation causes some dehydrochlorination of the chloroethylhalobenzene to give chlorostyrenes which polymerise in the still.

L. Williams in British Pat. No. 986634 overcame this important disadvantage by finding that rapid monobromination in the alkyl group of benzene homologues at room temperatures occurs by

irradiation of an equimolar solution of bromine and the hydrocarbon with light in the infrared and visible spectrum.

Under these conditions, no addition to the nucleus occurs. Further, although one bromine atom is readily introduced, the introduction of a second bromine atom is very much slower and it is thus possible to carry monobromination of the alkyl group to 100% merely by mixing the theoretical amount of bromine with the hydrocarbon prior to irradiation.

The main disadvantages common to all the processes described are:

a) the relatively high dehydrohalogenation temperatures which must be used results in carbonization of the catalysts. This carbonization results in the need for frequent regeneration or replacement of the catalyst with consequent interruption of the process and economical problems due to catalyst waste and excessive reactor handling.

b) the vinylhalobenzenes produced cannot be prepared in a pure state by this process, because as described in all works, there is always a requirement of redistillation, aqueous hydrobromic acid separation and/or neutralization washings in order to attempt to attain high purity of vinylhalobenzene, all these operations being time and fuel consuming.

c) the high temperature required in these processes necessitate the use of special equipment because of the corrosive nature of the halogen containing compound.

d) most catalysts when applied to haloethylhalobenzene exhibit the serious drawback of causing the formation and deposition on the catalyst of a tarry substance which most probably is a polymer formed from the monomeric halostyrene that is itself the primary product of the catalytic dehydrohalogenation. This polymer formation cannot be avoided even when

a large excess of an inert diluent, such as steam or nitrogen, is fed together with the haloethylhalobenzene. This polymer deposition rapidly reduces the catalyst activity and frequent regeneration is required. If the catalyst is rejected after brief runs, recharging with fresh catalyst becomes a very costly operation.

e) if a diluting inert gas is used, as seen in all processes described, the contact time of reactant with catalyst is decreased and large amounts of unreacted reactant appear together with the product. A fractionating distillation in a vacuum is then an unavoidable step, still further increasing losses of the desired product.

To eliminate the deposition of the above mentioned tarry-by products on the fixed bed of the catalyst, a process is described in French Pat. No. 1576909, wherein the active catalyst as well as reaction medium comprises certain molten salt mixtures, and in particular mixtures containing bivalent metal chlorides, in combinations with other salts which decrease the melting point of the salt mixture. While, according to this disclosure it is possible to obtain, for example, high yields of vinyl chloride by elimination of hydrogen chloride from ethylene dichloride, the process produces poor yields of monomers in the case of heavy, relatively nonvolatile substrates.

An improvement of the above process was described by G. Berger et al. in U.S. Pat. No. 3737469 which describes a process whereby α and/or β-bromoethylbromobenzene was passed over molten salts in the presence of an aliphatic alcohol to produce bromostyrene and an alkylbromide at a temperature range of 250°C-500°C. In said Patent it is disclosed that when a "reactive diluent" instead of an inert gas, is used in conjunction

with the substrate and is passed through the molten salt medium, a substantially complete conversion of the substrate is achieved in a single pass, the term reactive designating a suitable substance which acts as acceptor for the hydrogen halide which is split during the reaction.

More recently, U.S. Pat. No. 3980722 claims a process for producing bromostyrene and lower alkylbromide by pyrolyzing β-bromoethylbromobenzene in the presence of a lower alcohol at a temperature in the range of 400-500°C without the use of catalyst.

These latter processes require high temperature and give conversions rates which could be improved upon. The high temperature required for the reaction necessitates the use of special equipment because of the corrosive nature of the halogen containing compound.

One of the latest patents which deals with the preparation of ring halogenated styrenes is U.S. Pat. No. 4292453. In this Patent ring halogenated β-bromoethylbenzenes are converted to ring halogenated styrenes monomers by reacting heterogeneously the β-bromoethylhalobenzene with strong aqueous alkali base in the presence of a phase transfer catalyst. This process is not feasible from an economical point of view due to the high cost of raw materials, namely NaOH or KOH, that are used in large excess, and the phase transfer catalyst. Other disadvantages are the difficult preparation of β-bromoethylhalobenzene by anti-Markovnikoff attack of HBr on styrene, and the phase transfer catalyst destruction in basic medium.

In accordance with the present invention there is now provided a simple process for the ready preparation of ring halogenated styrene monomers, i.e., mono, di or tri fluoro, chloro or bromo styrenes in high yield and purity from inexpensive and readily available raw materials.

Thus, the present invention provides a process for preparing a ring halogenated styrene monomer comprising heating an α-bromoethylhalobenzene at a temperature in the range of about 100-250°C in the presence of a polymerization inhibitor and with or without a catalyst whereby a corresponding ring halogenated styrene and hydrobromic acid are formed. Preferably said ring halogenated styrene is separated from the remaining reactants and products by distillation as formed, and said process is carried out in a distillation column under vacuum pressure.

The process may be carried out under a vacuum pressure of about 1.5-1000 mbar and preferably is carried out under a vacuum pressure of about 10-150 mbar and at a temperature of about 130-180°C.

The process is preferably run without solvent particularly for the preparation of mono and dibromostyrene, however solvents may be used when desired.

Suitable solvents are nonvolatile solvents, for example polyethylene glycols and any other reactive solvents that may react with the eliminated hydrogen bromide, or on the other hand nonreactive solvents.

This process may be carried out non-catalytically in the presence of a polymerization inhibitor as stated, however the rate of formation of the halostyrene from the α-bromoethylhalobenzene can be enhanced by the use of catalytic amounts of soluble nucleophiles such as quaternary amonium or phosphonium salts.

Polymerization inhibitors are well known in the polymer art, however, heretofore, despite the wide-felt need for an effective and simple process for preparing ring-halogenated styrene monomers, it has neither been taught nor suggested that the introduction of well-known polymerization inhibitors together with an α-bromoethylhalobenzene under comparatively mild heating conditions of 100° to 250°C would result in the formation of the desired styrene monomers in high purity and yield.

Various types of polymerization inhibitors have been tested and found to be effective in the presently claimed process such as phenol derivatives, hydroxy phenols and sulfides and thus, a wide choice is available to the man in the art carrying out the present process.

As polymerization inhibitors of the phenol derivative type one can mention:
2,6-di-tert.-butyl-4-hydroxy-toluene (BHT),
3-tert-butyl-4-hydroxy-anisole (BHA); and
picric acid.

Possible hydroxyphenols include: hydroquinone, benzoquinone and 4-methyl, 4-tert. butyl, 4-tert.-amyl, 4-octyl, 4-phenyl, cyclohexyl, 4-benzyl, 3-methyl, 3,5-di-tert.-butyl and 3-phenyl catechols.

Among the sulfides may be mentioned thiopropionates such as dilauryl, 3,3'-thiodipropionate and sulfur itself.

Of the above mentioned polymerization inhibitors especially preferred is a polymerization inhibitor selected from the group consisting of di-tert/-butyl-hydroxytoluene, 2,6-di-tert.-butyl 4-hydroxy anisole, 3,5-tert.-butyl-catechol and diluaryl-3,3'-thiodipropionate.

The amount of polymerization inhibitor to be used will vary and as little as 0.001% w/w or as much as 20% or more w/w may be used, although said process is preferably carried out in the presence of about 0.01 to 5.0% w/w polymerization inhibitor and an amount of between about 0.05 and 1% w/w is especially preferred.

As indicated the process proceeds with high yields in the absence of a catalyst which is not necessary for the reaction, however, the presence of catalysts enhance the reaction rate and this procedure can preferably be used to obtain higher reaction rates in the styrene derivative production.

Various types of catalysts in the thermal dehydrohalogenation of α- and β-bromoethylhalobenzenes have been proposed but it has now been found that catalysts which are known in the art as phase transfer catalysts are especially effective in the present process.

As dehydrobromination catalysts one can mention for example quaternary ammonium salts of long and short amines or quaternary phosphonium salts which may be represented by the formula:

$$N^+R_1R_2R_3R_4X^-$$

$$P^+R_1R_2R_3R_4X^-$$

wherein $R_1$, $R_2$, $R_3$ and $R_4$ may be the same of different and is hydrogen alkyl, aryl, aralkyl, hydroxyalkyl or an alkoxyalkyl group and X is a nucleophilic anion.

Preferred anions of these catalysts include $Cl^-$, $I^-$, $Br^-$, $CN^-$, $S^{2-}$, $HS^-$, $NO_3^-$, $NO_2^-$, $ClO_4^-$, $ClO_3^-$, $RSO_3^-$ and $RCOO^-$ wherein R is a known per organic group for such anions, e.g. toluene, naphthalene, acetate, salicylate etc.

The catalyst may also be used in a bounded form with any support well known to the art.

The amount of catalyst used will vary and may be as little as 0.001 % wt. This amount can be increased depending on the desired reaction rate, and it has been found that amounts between 0.5 - 5% wt are preferred. Of the above mentioned catalysts especially preferred are tetrabutylammonium bromide, aliquat 336, triethylbenzylammonium chloride and tetrapropylammonium bromide.

Preferably the α-bromoethylhalobenzene used as the starting material in the present claimed process step is prepared by bromination of the corresponding ethylhalobenzene, said bromination being carried out by mixing said ethylhalobenzene with the stoichiometric amount of bromine, and exposing the mixture to light in the visible and/or ultra violet spectrum at a temperature that ranges between about 0 - 100°C, or higher.

In said preparation step it is especially preferred to use visible light as the radical initiator.

Two practical methods for carrying out said preparation step involve:

a. a continuous system where equivalent amounts of bromine and ethyl halobenzene are mixed and dropwise passed through an irradiated tube at ambient or elevated temperatures;

b. a semicontinuous system where bromine is added dropwise to an irradiated flask containing ethyl halobenzene also at ambient or elevated temperatures.

The major advantages of using visible light is the fact that quantitative conversions of ethylhalobenzene to α-bromoethylhalobenzene can be obtained without any side products.

An alternative but less attractive possibility for preparing the α-bromoethylhalobenzene is to use a radical initiator such as benzoyl peroxide or αα'-azo-isobutyronitrile. In such a system bromine is added to ethylhalobenzene in a semicontinuous system as described above. The disadvantage of this method is that conversions of only 60-70% can be obtained without appearance of side products. This lower conversion makes the process more difficult because of the need of prior distillation of the unreacted ethylhalobenzene.

The ethylhalobenzene used as the starting material in the above-described step is preferably prepared by ring halogenation of ethylbenzene catalyzed by $FeCl_3$ or other Lewis acids known to the art. The reaction is preferably effected in the dark and at a temperature which preferably does not substantially exceed 50-55°C.

Hydrogen halide produced as a consequence of the reactions is conveyed to a central unit to recover it in a pure anhydrous form.

Thus the present invention provides for a simple three step process for the preparation of halostyrenes of the general formula I

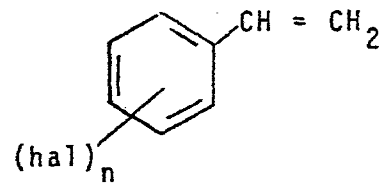

(I)

wherein n is 1-3 and hal represents a bromo, chloro or fluoro group comprising:

a) reacting ethylbenzene and halogen in the presence of a Lewis acid in order to introduce one or more halogen atoms in the aromatic nucleus.

b) brominating the side chain of the resulting ethyl halobenzene by means of irradiation; and

c) heating the resulting α-bromoethylhalobenzene of the general formula II

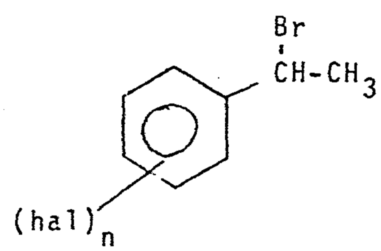

(II)

wherein hal and n are as defined at a temperature in the range of about 100-250°C in the presence of a polymerization inhibitor alone or in the presence of one of the above mentioned catalysts, the halostyrene being distilled as formed and the HBr being removed by vacuum.

More specifically, the present invention thus provides for a three step simple process for the ready preparation of vinylhalobenzenes in high yield and purity from cheap and readily available raw materials which process comprises the following steps:

1) Preparation of ethylhalobenzene by ring halogenation of ethylbenzene catalyzed by $FeCl_3$ or other Lewis acids known to the art. The reaction in this step is preferably effected in the dark and at a temperature which preferably does not substantially exceed 50-55°C.

Hydrogen halide produced as a consequence of the reaction is preferably conveyed to a central unit to recover it in a pure anhydrous form;

2) Preparation of α-bromoethylhalobenzene by mixing theoretical amounts of ethylhalobenzene and bromine in the dark, and irradiating the mixture while passing through a transparent glass tube where the reaction proceeds. The reaction mixture which is irradiated is maintained at a temperature range of 1-100°C and preferably 0-50°C. As in the first step, hydrobromic acid is recovered in a pure anhydrous form. The main product is α-bromoethylhalobenzene and there is no need for distillation; and

3) Dehydrobromination of α-bromoethylhalobenzene by heating it, preferably under the appropriate vacuum, in the presence of a polymerization inhibitor with or without a catalyst. The temperature range is 100-250°C and preferably 130-180°C the inhibitor amount lies between 0.01 and 5%, and the catalyst lies between 0.01 and 10% and preferably between 0.1 and 5%, depending on the desired reaction rate.

The bromoethylhalobenzene is preferably added to the hot zone reaction at a similar speed as the halostyrene is distilled at the top of the column to ensure a minimum residence time of the bromoethylhalobenzene and the halostyrene at high temperatures. The finished styrenes are of good purity and usually there is no need for redistillation in order to purify the styrene monomer.

The optimum temperature, vacuum pressure and inhibitor amount for the preparation of any one ring halogenated styrene can readily be determined in each case within the general ranges given above.

Compared with other processes involving catalytic dehydrohalogenation, the present process has the following advantages:

1) Readily available raw materials are used;

2) The lower temperatures available in this dehydrobromination simplify the energy requirements of the process;

3) Yields of all three of the above described steps are nearly complete, since all the α-bromoethylhalobenzene isomers dehydrobrominate simultaneously;

4) The resulting halogenated styrenes are substantially pure and contain no aliphatically bound bromine, so they may be used as condensed from the heating unit without any separation of aqueous dilute hydrogen halide solution, neutralization, redistillation or any other purification operation; this fact simplifies to a minimum reactor handling and material losses, saving energy, money and time;

5) There is no need of reactant dilution in an inert or reactive diluent, minimizing and simplifying the reactor system;

6) Almost complete elimination of polymer formation due to inhibitor presence;

7) Production rates can be controlled by the use of variable amounts of dehydrobromination catalyst.

While the invention will now be described in connection with certain preferred embodiments in the following examples it will be understood that it is not intended to limit the invention to these particular embodiments. On the contrary, it is intended to cover all alternatives, modifications and equivalents as may be included within the scope of the invention as defined by the appended claims.

Thus, the following examples which include preferred embodiments will serve to illustrate the practice of this invention, it being understood that the particulars shown are by way of example and for purposes of illustrative discussion of preferred embodiments of the present invention only and are presented in the cause of providing what is believed to be the most useful and readily understood description of formulation procedures as well as of the principles and conceptual aspects of the invention.

EXAMPLE 1

150 gm of crude α-bromoethylmonobromobenzene was mixed with 0.075 gm (500 ppm) butylated hydroxy toluene (BHT) in a 250 ml three necked flask equipped with a packed distillation column, thermometer, and mechanical stirrer. The mixture was heated at 150°C under vacuum (25-35 mbar) and 03.6 gm (90.1% yield) 98% pure bromostyrene was collected as the distillate.

EXAMPLE 2

The procedure of Example 1 was repeated using 0.075 gm dilauryl 3,3' thiopropionate as polymerization inhibitor at 170°C with vacuum of 45 mbar. 96.4 gm (92.8% yield) bromostyrene was collected as distillate.

EXAMPLE 3

The procedure of Example 1 was repeated using 1.5 gm hydroquinone as polymerization inhibitor at 25 mbar at 135°C. 82.1 gm (78.9% yield) bromostyrene was collected as distillate.

EXAMPLE 4

The procedure of Example 1 was repeated with 150 gm of crude α-bromoethylmonofluorobenzene resulting in an 88% yield of fluorostyrene of 95% purity as distillate.

EXAMPLE 5

The procedure of Example 1 was repeated using 1.5 gm picric acid as polymerization inhibitor. 84.8 gm (81.5% yield) bromostyrene was collected as distillate.

EXAMPLE 6

The procedure of Example 1 was repeated using α-bromoethyl monochlorobenzene as reactant. 88.4 gm (93.4% yield)chlorostyrene was collected as distillate.

EXAMPLE 7

The procedure of Example 1 was repeated using 150 gm of α-bromoethyldibromobenzene as reactant and 1.5 gm BHT as inhibitor.103.0 gm (89.5%) yield of dibromostyrene was collected as the distillate.

EXAMPLE 8

The procedure of Example 1 was repeated using 150 g of α-bromoethyldichlorobenzene as reactant and 1.5 gm BHT as inhibitor. 93.9 gm (91.2% yield) of dichlorostyrene was collected as the distillate.

EXAMPLE 9

40 gm of crude α-bromoethylbromobenzene was mixed with 0.2 gm BHT in a 250 ml three necked flask equipped with a packed distillation column, mechanical stirrer, thermometer and dropping funnel. The flask was heated to boiling (180°C) under a vacuum of 50 mbar. 650 gm of reactant were added dropwise as 98% pure bromostyrene was collected as distillate. In all 454 gm (95% yield) of bromostyrene was collected.

EXAMPLE 10

106 gm (1.0 mol) ethylbenzene and 0.5 gm $FeCl_3$ were mixed in a 500 ml three necked flask and heated to 50°C. 53 ml of molecular bromine (1.0 mol) was added dropwise for three hours while the mixture was stirred. After an additional hour the organic phase was washed once with water and once with dilute $Na_2CO_3$ solution. The organic phase was dried by azeotropic toluene distillation and the crude ethylbromobenzene was distilled under vacuum yielding 179 gm (97.8% yield) of 99% pure ethylbromobenzene.

The ethylbromobenzene was mixed with 52 ml of molecular bromine as fed dropwise thorugh an irradiated condensor at 15°C. 250.4 gm (97% yield) of crude α-bromoethylbromobenzene was collected. The crude product was then pyrolized in the presence of 0.125 gm BHT under a 30 mbar pressure in a 500 ml three necked flask equipped with a distillation column, mechanical stirrer and thermometer. 165 gm (0.90 mol) of 98% pure bromostyrene was collected as distillate.

EXAMPLE 11

1700 grs of crude α-bromoethylbromobenzene was added dropwise to a 1 liter three necked flask equipped with a distillation column mechanical stirrer and thermometer containing 200 gr of α-bromoethylbromobenzene, 5 grs of BHT, and 15 grs of tetrabutyl ammonium bromide at 150-100°C and 30 Mbars. 1211 grs (92% yield) of 98.2% pure bromostyrene was collected as the distillate.

EXAMPLE 12

The same procedure as in Ex. 11 was used using 15 grs of tetramethylammonium bromide as dehydrobrominating catalyst. 1200 grs (91% yield) of pure bromostyrene was collected as distillate.

EXAMPLE 13

The procedure of example 11 was repeated using 2000 grs of α-bromoethyldibromobenzene, 15 grs of tetrabutylammonium-bromide and 10 grs of BHT.

940 grs of dibromostyrene (88% yield) was collected as distillate.

It will be evident to those skilled in the art that the invention is not limited to the details of the foregoing illustrative examples and that the present invention may be embodied in other specific forms without departing from the essential attributes thereof, and it is therefore desired that the present examples be considered in all respects as illustrative and not restrictive, reference being made to the appended claims, rather than to the foregoing description, and all changes which come with the meaning and range of equivalency of the claims are therefore intended to be embraced therein.

CLAIMS:

1. A process for preparing a ring-halogenated styrene monomer comprising heating an α-bromoethyl halobenzene at a temperature in the range of about $100^o$ - $250^oC$, preferably about 130° - 180°c, in the presence of a polymerization inhibitor whereby a corresponding ring-halogenated styrene and hydrobormic acid are formed.

2. A process according to claim 1 comprising gradually introducing said α-bromoethyl halobenzene, the polymerization inhibitor and a catalyst to a reaction zone, wherein the mixture is heated to a temperature between 130-$180^oC$ while distilling at the appropriate vacuum pressure the corresponding ring halogenated styrene from the mixture as it is formed, the rate at which the α-bromoethyl halobenzene reactant is introduced to the reaction being controlled so as to prevent substantial aaccumulation of the same in the hot reaction mixture.

3. A process according to claim 1, wherein said ring-halogenated styrene is separated from the remaining reactants and products by distillation, preferably the process being effected in a distillation column.

4. A process according to claim 1 or claim 3, wherein said process is carried out in the presence of a catalyst.

5. A process according to claim 2 or claim 4, wherein said catalyst is a quaternary ammonium or quarternary phosphonium salt.

6. A process according to any one of claims 2,

4 or 5, wherein said process is carried out in the presence of about 0.01 to 10% w/w of catalyst.

7. A process according to any one of claims 1 to 6, wherein said process is carried out under a vacuum pressure of about 1.5 -1000 mbar, preferably about 25-75 mbar.

8. A process according to any one of claims 1 to 7, wherein said process is carried out in the presence of about 0.001-20% w/w polymerization inhibitor, preferably about 0.05-1% w/w polyermization inhibitor.

9. A process according to any one of claims 1 to 8, wherein said polymerization inhibitor is a phenol, catechol or sulfide derivative.

10. A process according to claim 9, wherein said polymerization inhibitor is di-tert.-butyl-hydroxytoluene, 3-tert-butyl hydroxy anisole, tertbutyl catechol or dilauryl-3,3'-thiodipropionate.

11. A process according to any one of claims 1 to 10, wherein said α-bromoethyl halobenzene is α-bromoethyl chlorobenzene, α-bromoethyl bromobenzene, α-bromoethyl fluorobenzene, α-bromoethyl dichlorobenzene or α-bromoethyl dibromobenzene.

12. A process according to any one of claims 1 to 11 in which the α-bromoethyl halobenzene is prepared by bromination of the corresponding ethyl halobenzene, said bromination being carried out by mixing said ethyl halobenzene with the stoichiometric amount of bromine, and exposing the mixture to light in the visible and/or infrared spectrum at a

temperature that ranges between about 0-80$^{o}$C.

13. The use of a ring-halogenated styrene monomer which has been prepared by a process according to any one of claims 1 to 12 in the production of a polymer, optionally with other monomers.

14. The use of a polymer produced from a styrene monomer which has been prepared by a process according to any one of claims 1 to 12 in the production of a molded article, a latex paint, a synthetic rubber, a coating or as an intermediate in the plastics industry.